Europäisches Patentamt

European Patent Office

Office européen des brevets

(11) Publication number: **0 323 785**
**A1**

## EUROPEAN PATENT APPLICATION

(21) Application number: 88403353.1

(22) Date of filing: 28.12.88

(51) Int. Cl.4: **G01N 33/564 , G01N 33/53 , G01N 33/543**

(30) Priority: 28.12.87 US 138809

(43) Date of publication of application:
**12.07.89 Bulletin 89/28**

(84) Designated Contracting States:
**AT BE CH DE ES FR GB GR IT LI LU NL SE**

(71) Applicant: **THE REGENTS OF THE UNIVERSITY OF CALIFORNIA**
**2199 Addison Street**
**Berkeley California 94720(US)**

(72) Inventor: **Weisbart, Richard H.**
**9621, Lockford Street**
**Los Angeles California 90035(US)**

(74) Representative: **Warcoin, Jacques et al**
**Cabinet Régimbeau 26, avenue Kléber**
**F-75116 Paris(FR)**

(54) IGG rheumatoid factor test.

(57) An improved sensitive test for detecting rheumatoid factors is provided by employing ovine immunoglobulin G as the ligand in conventional immunoassay protocols. Greater specificity and sensitivity is achieved as compared to present day assays, as well as provided for a correlation between disease activity and the level of rheumatoid factor.

EP 0 323 785 A1

## IGG RHEUMATOID FACTOR TEST

### INTRODUCTION

#### Technical Field

The subject field is the detection of rheumatoid factor.

#### Background

Rheumatoid arthritis (RA) is characterized clinically by the chronic inflammatory destruction of peripheral joints and seriologically by the presence of antiglobulin antibodies called rheumatoid factors (RF). Although RF may be of the IgM, IgG or IgA class of antibodies, most clinical assays primarily measure IgM RF by its capacity to agglutinate latex particles coated with aggregated human IgG. In spite of its prominent association with RA, RF measured by latex agglutination is frequently present in the sera of many non-RA patients including those with viral, parasitic and bacterial infections, cirrhosis, neoplasia and even in the aged. Moreover, IgM RF measured by latex agglutination correlates poorly with RA disease activity. Rheumatoid factors of the IgG class are of special interest because they can self-associate to form potentially pathogenic immune complexes. However, IgG RFs are technically difficult to measure and assays for their detection have not been available for clinical use. Characterization of IgG RFs in RA sera shows binding at or near the IgG $F_c$ binding site of Staphylococcus aureus protein A (SpA), suggesting that the IgG RFs have structural homology with SpA. Sub-class distribution of IgG RF is a predominance of $IgG_4$ antibodies in both the sera and synovial fluids of RA patients. The binding of RF in RA sera to heterologous mammalian IgG is consistent with its binding specificity for SpA and is a distinctive feature which tends to distinguish it from RFs which occur in the sera of non-RA patients. ·

The binding of human IgG RF to horse IgG was shown to provide a more sensitive assay for detecting RF in the sera of rheumatoid arthritis patients. However, the RA disease specificity of IgG RFs which bind different mammalian IgGs has not been evaluated. It is of substantial interest to improve techniques for detecting RFs by providing new reagents, new protocols, and new insights in relationships between binding and RA.

#### Relevant Literature

Nardella et al., J. Exp. Med. (1985) 162:1811 report that IgG RFs and Staphylococcal protein A bind to a common molecular site on IgG. Cohen et al., J. Immunology (1987) 139:1466 report that IgG RF show a predominance of $IgG_4$ antibodies in RA patients. Larsen, Rheumatoid Factor In: Textbook of Rheumatology. (Kelly et al., eds.) W.B. Saunders Co., PA, 1981. p685.; and Pope and McDuffy, J. Lab. Clin. Med. (1981) 97:842-853 discuss the binding of RF in RA serum to heterologous mammalian IgG. That reference also reports that horse IgG provides a more sensitive assay for detecting RF in rheumatoid arthritis patient sera.

### SUMMARY OF THE INVENTION

Ovine IgG is employed as a ligand for the detection of rheumatoid factor (RF) in rheumatoid arthritis patients. A variety of conventional assays may be employed for detection of complex formation between RF and the ovine IgG ligand.

### DESCRIPTION OF THE SPECIFIC EMBODIMENTS

An improved method and compositions are provided for detecting human RF in a human host suspected of having rheumatoid arthritis (RA). The method employs ovine IgG as the ligand to which the RF binds. Various protocols and reagents may be employed for detecting complex formation between the RF and the ovine IgG.

The ovine IgG may be obtained in any convenient manner and in accordance with conventional techniques for isolating IgG from mammalian blood. The IgG may obtained from commercial sources including Organon Teknika, West Chester, Pennsylvania. Of particular interest is sheep or goat IgG, more particularly sheep.

Various protocols may be employed for detecting binding of the RF in blood or a blood derivative to the ovine IgG. Particularly, heterogeneous assays will be employed. The labels may include radioisotopes, enzymes, fluorescers, chemiluminescers, particles, or other label which provides for the desired level of sensitivity. Enzymes which may be employed include hydrolases and oxidoreductases, such as alkaline phosphatase, $\beta$-galactosidase, glyceryl-3-phosphate dehydrogenase, malate dehydrogenase, glucose-6-phosphate dehydrogenase, horse radish peroxidase, glucose oxidase, uricase, xanthine oxidase, etc. Fluorescers which may be employed include phycobiliproteins, fluorescein, dansyl, rhodamine, etc. Radioisotopes which may be employed include $^{32}P$, $^{14}C$, $^3H$, $^{35}S$, etc.

Conveniently, sandwich assays may be employed, where the ligand is bound to a substrate, either covalently or non-covalently. Various activated substrates are available, having functional groups which will bind to the ovine IgG. Illustrative groups include activated carboxyl groups, imino groups, aldehydes, etc. Alternatively, the ligand may be coated onto a plastic surface, e.g. polystyrene, and heated and dried so as to form a strong non-covalent bond.

The sample may then be contacted with the substrate bound ligand. The blood sample may have been subject to prior treatment, such as removal of red blood cells to provide for serum or plasma, citration, dilution with buffer, or the like. Usually, the sera will not be diluted more than about 100-fold, frequently not more than about 50-fold.

Various buffers may be employed, being selected to be compatible with the assay. Buffering agents include Tris, MOPS, phosphate, borate, carbonate, etc. The particular buffer will be primarily a matter of arbitrary choice, although one buffer may be preferred as compared to another in a particular assay protocol. Generally, the buffer will range from about 10 to 400 mM and the pH will generally range from about 5 to 11, more usually from about 6 to 10. Other components may include inert proteins, such as bovine serum albumin, to reduce non-specific binding, the added proteins usually being present in not more than about 2%.

The sample, as pre-treated, may then be added to the solid substrate- or support-bound-ligand, conveniently at or about room temperature, and incubated for sufficient time for complex formation to occur. Once complex formation has occured, antibodies, either polyclonal or monoclonal, specific for human RF may be added to form a second reaction mixture. Such antibodies are normally labeled. The second reaction mixture is incubated for sufficient time for the labeled antisera or monoclonal antibodies to bind and non-specifically bound labeled antibodies washed away, conveniently by repeated washings, usually not more than about three washings. The label may then be detected in accordance with its nature.

For a radioisotope, the supernatant will be separated from the solid substrate and the radioactivity of either the solid substrate or the supernatant or both determined. For an enzyme, after separating the supernatant from the solid substrate, a substrate solution may then be added to either the solid substrate or the supernatant and the production of product determined as indicative of the amount of enzyme present. For fluorescers, the substrate or supernatant may irradiated with excitory light and the amount of fluorescence determined.

A large number of assay protocols are found in the literature. See, for example, U.S. Patent Nos. 3,654,090; 3,850,752; 3,996,345; 4,134,792; 4,208,479 and 4,331,647. These protocols may be adapted to the subject assay for determination of RF associated with rheumatoid arthritis.

The following examples are offered by way of illustration and not by way of limitation.


## EXPERIMENTAL


### Patients Studied

Rheumatoid factors were measured in the sera of 112 patients who fulfilled American Rheumatism Association criteria for the diagnosis of classical or definite rheumatoid arthritis (Ropes et al., 1958 Revision of Diagnostic criteria for rheumatoid arthritis. Arthritis Rheum. (1959) 2:16-20). RA disease activity was evaluated at the time serum was obtained in 25 patients by a joint count consisting of the physician's objective evaluation of the number of swollen or tender joints, the patient's assessment of pain, and a quantitative morning stiffness in minutes or hours. Each parameter was rated on a scale of 0 (inactive) to 5 (most active) and an index of disease activity was determined as the sum of the three parameters evaluated. Sera from non-rheumatoid arthritis patients was screened for the presence of IgM RF and found in 32 subjects including 10 patients with cirrhosis or hepatitis, 4 with malignancy, 3 with ankylosing spondylitis, 2 with bacterial endocarditis, 2 with gout, 1 with Crohn's disease, and in 10 geriatric subjects. Sera from these individuals were then examined for the presence of IgG RF.

Rheumatoid Factor Assays

IgM RF was measured by observing the agglutination of latex beads coated with aggregated human IgG by human sera. Tests were performed with RA latex kits purchased from DIFCO Laboratories, Detroit, Michigan. Patients sera were diluted 1:20 with glycine buffer to which an equivalent amount of latex reagent was added. After thorough mixing, the latex beads were observed for agglutination. Positive and negative controls were included in each assay. Quantitative results were obtained by assaying sera in 2-fold serial dilutions and observing the greatest dilution at which agglutination occurred.

IgG rheumatoid factors were measured by an enzyme-linked immunosorbent assay (ELISA). Ninety-six well microtiter plates were coated with purified IgG from seven mammalian species including rabbit, horse, mouse, guinea pig, sheep, and goat, and with human IgG Fc as follows: IgG (10 $\mu$g/ml) in 0.06 M carbonate buffer. pH 9.6, was incubated overnight at 4°C in 96-well plates. Sera were assayed at a dilution of 1:100, and human IgG was detected by peroxidase-conjugated sheep anti-human IgG antibodies specific for the F-(ab')$_2$ of IgG. Peroxidase was detected by the enzymatic conversion of 2,2' azino-di(3-ethylbenzothiaz olinsulfone-6) (diammonium salt) measured spectrophotometrically and expressed as absorbance at 414nm. Positive results for IgM and IgG RF assays were established as 2 S.D. above the mean for sera from 20 healthy individuals.

Results

Serum IgM and IgG Rheumatoid Factors in Rheumatoid Arthritis Patients

IgM rheumatoid factor activity was detected in 59 of 112 (52.6%) RA patients at a serum dilution of 1:40.

Table 1

| Serum IgM and IgG Rheumatoid Factors in Rheumatoid Arthritis Patients | | |
|---|---|---|
| | No. Positive[*] (Total = 112) | % Positive |
| IgM RF (latex agglutination) | 59 | 52.6 |
| IgG RF (ELISA) to mammalian IgG: | | |
| Horse | 81[+] | 72.3 |
| Rabbit | 78[+] | 69.6 |
| Sheep | 72[+] | 64.3 |
| Goat | 70[+] | 62.5 |
| Human IgC Fc | 48 | 42.9 |
| Guinea Pig | 43 | 39.3 |
| Mouse | 33 | 29.5 |

[*] IgM RF was positive at titers ≥1:40; IgG RF with absorbance values greater than 2 S.D. above the mean for 30 healthy control subjects were considered positive.
[+] The incidence of IgG RF responses was significantly greater than the incidence of IgM RF responses (p<0.01).

The incidence of IgG RF to human IgG Fc in RA was slightly lower but not significantly different from IgM RF, occurring in 48 of 112 RA patients (42.9%). However, IgG RF to several of the other mammalian immunoglobulins provided a more sensitive assay for RA than either the IgM RF assay by latex agglutination or the IgG RF assay employing human IgG Fc. The mammalian IgGs which were more sensitive than human IgG Fc for the detection of IgG RF in RA patients included horse, rabbit, sheep, and goat IgG which gave positive results in 62-72% of RA patients (Table 1).

Serum IgM and IgG Rheumatoid Factors in Non-Rheumatoid Arthritis Patients

Sera were identified with IgM RF titers of 1:40 or greater from 32 non-rheumatoid arthritis patients. These sera were then assayed for IgG RF by ELISA using different mammalian immunoglobulins. IgG RF was found in only 7 to 15 of the 32 IgM RF-positive sera when assayed using different mammalian IgGs. The results demonstrate that all of the IgG RF assays were more specific for RA than the IgM RF latex agglutination test (Table 2). However, IgG RF to sheep, goat, guinea pig, and mouse IgG and to human IgG Fc were more specific for RA than IgG RF to horse and rabbit IgG.

Table 2

| Serum IgM and IgG Rheumatoid Factors in Non-Rheumatoid Arthritis Patients* | | |
|---|---|---|
| | No. Positive[+] (Total = 32) | % Positive |
| IgM RF (latex agglutination) | 32 | 100.0 |
| IgG RF (ELISA) to mammalian IgG: | | |
| Horse | 12 | 37.5 |
| Rabbit | 15 | 46.9 |
| Sheep | 8 | 25.0 |
| Goat | 8 | 25.0 |
| Human IgG Fc | 8 | 25.0 |
| Guinea Pig | 7 | 21.9 |
| Mouse | 8 | 25.0 |

* Patients included 10 with cirrhosis or hepatitis, 4 with malignancy, 3 with ankylosing spondylitis, 2 with subacute bacterial endocarditis, 2 with gout, 1 with Crohn's disease, and 10 geriatric patients (age >65) without apparent disease.
[+] Positive tests for IgM RF had a titer of ≥1:40, and IgG RF had absorbance values greater than 2 S.D. above the mean for 30 healthy control subjects.

## Sensitivity and Specificity of IgG RF Assays for Rheumatoid Arthritis

The relative sensitivity and specificity of each IgG RF assay was calculated with respect to the IgM RF assay.

Table 3

| Sensitivity and Specificity of IgG Rheumatoid Factors Binding Different Mammalian IgG Immunoglobulins* | | | |
|---|---|---|---|
| Mammalian IgG | Relative Sensitivity | Relative Specificity | Product Index |
| Horse | 1.37 | 2.67 | 3.66 |
| Rabbit | 1.32 | 2.13 | 2.82 |
| Sheep | 1.22 | 4.00 | 4.88 |
| Goat | 1.19 | 4.00 | 4.76 |
| Human IgG Fc | 0.81 | 4.00 | 3.24 |
| Guinea Pig | 0.75 | 4.57 | 3.43 |
| Mouse | 0.56 | 4.00 | 2.24 |

* The sensitivity and specificity of IgG Rfs binding different mammalian IgGs are given relative to the IgM RF by latex agglutination with a value of 1.00.

As shown in Table 3, horse IgG provided the most sensitive test for RA, but guinea pig IgG was the most specific. The product of the sensitivity and specificity was used to establish a product index indicating the overall best test based on sensitivity and specificity. Sheep IgG gave the best combined result; it provided an assay which was 1.2-fold more sensitive than the standard IgM RF by latex agglutination and 4.0-fold more specific. Thus, using sheep IgG to detect human IgG RF provided an assay which was nearly 5-fold better than standard IgM RF latex agglutination test. Only goat IgG gave results which were closely comparable to sheep IgG.

Correlation of IgM and IgG RF with Rheumatoid Arthritis Disease Activity

IgG RFs which bind mammalian IgGs have not been studied for their correlation with RA disease activity. Serum was obtained from 25 RA patients and examined for the presence of IgM RF by latex agglutination and IgG RF by ELISA using sheep IgG. The clinical characteristics of these patients are shown in Table 4.

Table 4

| Clinical Characteristics of Rheumatoid Arthritis Patients | |
| --- | --- |
| Number (F/M) | 25 (8/17) |
| Mean age (range) | 61 (31-75) |
| Duration (Yrs) disease (range) | 17 (1-47) |
| No. patients with nodules | 9 |
| No. patients receiving DMARDS* | 25 |

* Disease modifying antirheumatic drugs received included gold, hydroxychloroquine, D-penicillamine, azathioprine and methotrexate.

Most of the patients studied in a Veterans Administration Medical Center were male (17/25), with a mean age of 61 and an average disease duration of 17 years. Clinical assessment of disease activity included a joint count, evaluation of severity of joint pain, and a quantitation of morning stiffness. The titer of IgM RF by latex agglutination and the asorbance level of the IgG RF to sheep IgG by ELISA were correlated with the RA disease activity index as determined by the clinical parameters. IgM RF by latex agglutination did not correlate well with disease activity (0.35 compared to IgG RF to sheep IgG by ELISA ($r = 0.76$)). The Westergren erythrocyte sedimentation rate was determined in the same patients, but it did not correlate as well with RA disease activity ($r = 0.60$) as IgG RF.

It is evident from the above results, that ovine immunoglobulin G as a ligand provides for a substantially better test for the determination of rheumatoid factor than is presently available. A more accurate diagnosis can be achieved with fewer false positives. Thus, individuals will not be subjected to unnecessary therapeutic treatment, which has inherent hazards, where they are suffering from a different disease. In addition, the physician will not be mislead as to the cause of the problem, so that the proper disease may be diagnosed and treated. Conventional protocols may be employed to provide for a rapid and simple determination of the presence of RF factor.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

**Claims**

1. In a method for detecting rheumatoid factor as diagnostic of rheumatoid arthritis by identifying the presence of rheumatoid factor in blood, the improvement which comprises:
employed ovine IgG as the ligand for binding to rheumatoid factor.

2. A method according to Claim 1, wherein said ovine IgG is sheep IgG.

3. In a method for detecting rheumatoid factor as diagnostic of rheumatoid arthritis by identifying the presence of rheumatoid factor in a blood sample, by combining said sample with a ligand, and detecting the presence of complexes of said ligand and said rheumatoid factor, the improvement which comprises:
employing sheep IgG as the ligand for binding to rheumatoid factor.

4. A method according to Claim 3, wherein said ligand is bound to a surface and said detecting employs a labeled receptor specific for human IgG.

5. A method according to Claim 4, wherein said labeled receptor is enzyme labeled antibody.

6. A method according to Claim 5, including the additional step of separating said complexes from said sample before said detecting.

7. A kit for detecting rheumatoid factor which comprises:
ovine IgG bound to a solid surface; and labeled receptor for human IgG.

8. A kit according to Claim 7, wherein said ovine IgG is sheep IgG and said labeled receptor is labeled antibody to human IgG.

9. A kit according to Claim 8, wherein said labeled antibody is enzyme labeled antibody.

10. A kit according to Claim 7, wherein said solid surface is a microtiter well plate.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | WO-A-8 607 152 (BIOSTAR MEDICAL PRODUCTS, INC.) <br> * Page 4, line 13 - page 7, line 9; page 15, line 9 - page 16, line 24; page 18, line 11 - page 20, line 5; page 23, line 7 - page 24, line 22; claims 1-3 * <br> --- | 1-10 | G 01 N 33/564 <br> G 01 N 33/53 <br> G 01 N 33/543 |
| X,D | JOURNAL LAB. CLIN. MED., vol. 97, no. 6, 1981, pages 842-853; R.M. POPE et al.: "IgG rheumatoid factor: analysis of various species of IgG for detection by radioimmunoassay" <br> * Abstract; page 843, lines 11-45; page 844, table II * | 1-4,6-8 | |
| Y | IDEM <br> --- | 5,9,10 | |
| Y | EP-A-0 096 520 (AGROPRODUITS SA) <br> * Abstract; page 2, line 17 - page 6, line 22 * <br> --- | 5,9,10 | |
| X | CLINICAL CHEMISTRY, vol. 30, no. 6, June 1984, pages 880-883, Washington, US; I. GIAEVER et al.: "A new assay for rheumatoid factor" <br> * Page 880; page 883, column 1, lines 24-27 * <br> --- | 1 | TECHNICAL FIELDS SEARCHED (Int. Cl.4) <br><br> G 01 N |
| X,P | JOURNAL OF IMMUNOLOGICAL METHODS, vol. 108, 1988, pages 205-208, Elsevier Science Publishers B.V.; A. LARSSON et al.: "Chicken antibodies: a tool to avoid false positive results by rheumatoid factor in latex fixation tests" <br> * Page 205 * <br> ---       -/- | 1-3 | |

The present search report has been drawn up for all claims

| Place of search | Date of completion of the search | Examiner |
|---|---|---|
| THE HAGUE | 05-04-1989 | HITCHEN C.E. |

European Patent Office

**EUROPEAN SEARCH REPORT**

Application Number

EP 88 40 3353

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | AM. J. CLIN. PATHOL., vol. 74, no. 6, 1980, pages 776-784, American Society of Chemical Pathologists; S.P. HALBERT et al.: "A quantitative enzyme immunoassay for IgM rheumatoid factor using human immunoglobulin G as substrate" * Page 776 - page 777, column 1, line 6; page 781, column 2, lines 14-21; page 783, column 2, lines 3-17 * | 1,7 | |
| A | BIOLOGICAL ABSTRACTS, vol. 73, no. 9, 1982, page 6363, abstract no. 61209, Philadelphia, PA, US; T. PALOSUO et al.: "Immunoglobulin G rheumatoid factor: Detection by enzyme immunoassay in rheumatoid arthritis and normal subjects", & J. IMMUNOL. METHODS 47(2): 171-182, 1981 | 1 | |
| A | BIOLOGICAL ABSTRACTS, vol. 65, no. 6, 1978, page 3298, abstract no. 33728, Philadelphia, PA, US; C.L. CAMBIASO et al.: "Particle counting immunoassay (pacia): I. A general method for the determination of antibodies, antigens, and haptens", & J. IMMUNOL. METHODS 18(1/2): 33-44, 1977 | 1 | TECHNICAL FIELDS SEARCHED (Int. Cl.4) |

The present search report has been drawn up for all claims

| Place of search | Date of completion of the search | Examiner |
|---|---|---|
| THE HAGUE | 05-04-1989 | HITCHEN C.E. |